# EUROPEAN PATENT APPLICATION

(11) **EP 4 364 815 A1**
(43) Date of publication of application: **08.05.2024**
(21) Application number: 22833242.5
(22) Date of filing: 29.06.2022
(51) Int. Cl.: A61Q 1/02, A61Q 17/04, A61Q 19/00, C08F 220/26, A61K 8/06, A61K 8/81, A61K 8/891

(54) **COPOLYMER AND COSMETIC PRODUCT INCLUDING SAME**

(30) Priority: 30.06.2021 JP 2021109184
(71) Applicant: NOF Corporation, Shibuya-ku Tokyo 150-6019 (JP)
(72) Inventor: SASAKI, Ryo, Tokyo 114-0005 (JP); MASUBUCHI, Yuji, Tokyo 114-0005 (JP); MUNEKATA, Yuki, Amagasaki-shi, Hyogo 660-0095 (JP); MATSUMOTO, Shinnosuke, Amagasaki-shi, Hyogo 660-0095 (JP); TAGAMI, Yasunobu, Amagasaki-shi, Hyogo 660-0095 (JP)
(74) Representative: Petty, Catrin Helen
(86) International application number: PCT/JP2022/026079
(87) International publication number: WO 2023/277096

(57) **Abstract**

An object of the present invention is to provide a copolymer having both favorable dispersibility in water and high water repellency when formed into a coating film. Provided is a copolymer containing more than 20 mass% and less than 50 mass% of a structural unit represented by the following Formula (1) in the copolymer, more than 0 mass% and less than 40 mass% of a structural unit represented by the following Formula (2) in the copolymer, and a structural unit represented by the following Formula (3): 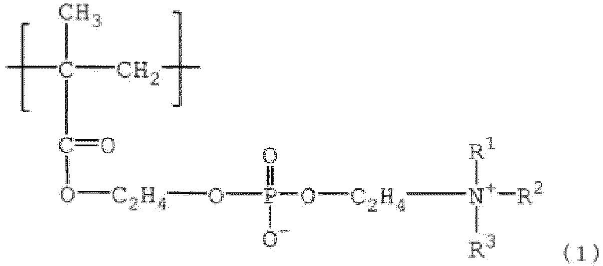 wherein R¹ to R³ each independently represent a linear or branched alkyl group having 1 to 30 carbon atoms which may be substituted, 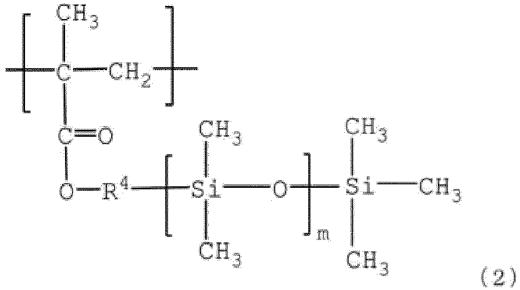 wherein R⁴ represents an alkylene group having 2 to 10 carbon atoms which may be substituted, and m represents an integer of 1 to 30, and 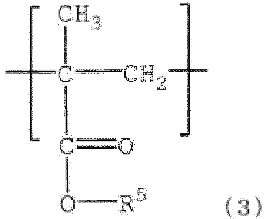 wherein R⁵ represents a linear or branched alkyl group having 1 to 30 carbon atoms which may be substituted.

## Description

### TECHNICAL FIELD

The present invention relates to a copolymer and a cosmetic containing the copolymer.

### BACKGROUND ART

Due to diversification of preference, there are various types of feeling required for cosmetics. In order to meet such requirements of consumers, there are various dosage forms of cosmetics. Among them, in water-based cosmetics and oil-in-water emulsified cosmetics (hereinafter, also simply referred to as "aqueous cosmetics"), since the continuous phase is composed of an aqueous component, such cosmetics have features in that (1) light and smooth spreading and a refreshing feeling of use are obtained, (2) production is relatively easy, and (3) they are hardly affected by temperature, when used as cosmetics.

On the other hand, in order to impart various functions, various polymers are blended in the cosmetic. For example, Lipidure (registered trademark of NOF CORPORATION), which is an MPC polymer containing methacryloyloxyethyl phosphorylcholine (hereinafter, abbreviated as "MPC") as a repeating unit, is known as a material excellent in moisture retention, skin adhesion, safety, and the like. MPCs having a phosphorylcholine group are excellent in biocompatibility (bioaffinity) and moisture retention, and are used as biocompatible materials, for example, as coating agents for artificial organs, biological membranes, and medical devices, drug delivery, and cosmetic blending components. On the other hand, as for silicones that are physiologically inert, very safe, and stable, which have properties essential for cosmetic raw materials, both solid silicones and liquid silicones (silicone oils) have been used in various applications in the field of cosmetics. As a cosmetic containing a polymer having both properties of a polymer having a phosphorylcholine group and silicone, for example, a cosmetic containing a polymer having an organopolysiloxane residue and a phosphorylcholine group has been proposed (Patent Literatures 1 to 3).

### Citation List

### Patent Literatures

Patent Literature 1: WO 2005/094758 A
Patent Literature 2: JP 2018-24591 A
Patent Literature 3: WO 2019/189608 A

### SUMMARY OF INVENTION

However, when the polymer disclosed in Patent Literatures 1 to 3 is blend in a system in which water is blended, it is difficult to stably blend the polymer. For example, in the cosmetic, an aqueous component is often blended in order to obtain a fresh feel, and there is a high demand for a functional material that can be stably blended in an aqueous system. On the other hand, in the cosmetic, durability against moisture such as sweat is also required, and a functional material having high water repellency when formed into a coating film is required.

Therefore, an object of the present invention is to provide a copolymer having both favorable dispersibility in water and high water repellency when formed into a coating film.

Another object of the present invention is to provide a cosmetic which has water resistance of a cosmetic film, suppresses secondary adhesion of the cosmetic to a mask or the like, had favorable makeup retention, and further has high stability over time.

The present invention is a copolymer containing more than 20 mass% and less than 50 mass% of a structural unit represented by the following Formula (1) in the copolymer, more than 0 mass% and less than 40 mass% of a structural unit represented by the following Formula (2) in the copolymer, and a structural unit represented by the following Formula (3): 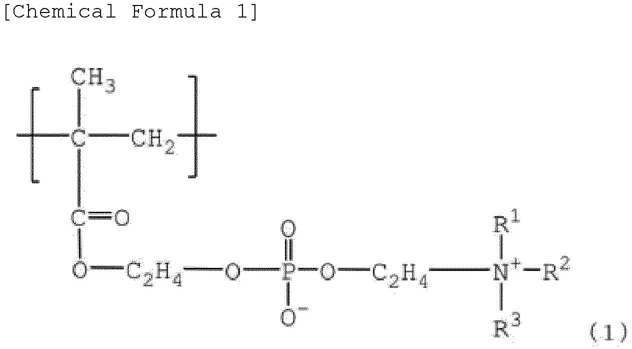 wherein R¹ to R³ each independently represent a linear or branched alkyl group having 1 to 30 carbon atoms which may be substituted, 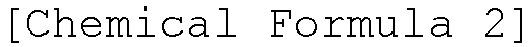 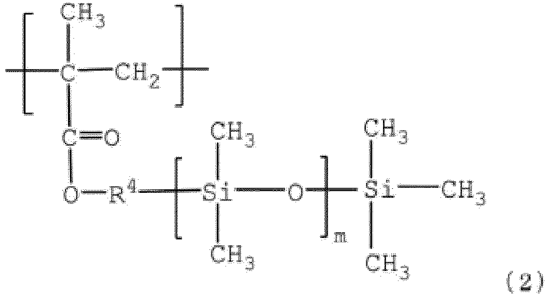 wherein R⁴ represents an alkylene group having 2 to 10 carbon atoms which may be substituted, and m represents an integer of 1 to 30, and 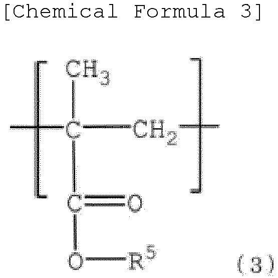 wherein R⁵ represents a linear or branched alkyl group having 1 to 30 carbon atoms which may be substituted.

### DESCRIPTION OF EMBODIMENTS

Hereinafter, embodiments of the present invention will be described. The present invention is not limited only to the following embodiments. In the present specification, the phrase "X to Y" indicating a range includes X and Y and means "X or more and Y or less". Unless otherwise specified, operations and measurements of physical properties and the like are performed under the conditions of room temperature (20 to 25°C)/relative humidity of 45 to 55% RH.

The present invention is a copolymer containing more than 20 mass% and less than 50 mass% of a structural unit represented by Formula (1) in the copolymer, more than 0 mass% and less than 40 mass% of a structural unit represented by Formula (2) in the copolymer, and a structural unit represented by Formula (3).

According to the copolymer of the present invention, dispersibility in water is favorable, and water repellency is high when the copolymer is formed into a coating film.

The copolymer of the present invention has high water repellency of a coating film to be formed and high dispersibility in water. Both high water repellency and favorable dispersibility in water are achieved by setting the ratio between the structural unit represented by Formula (1) and the structural unit represented by Formula (2) in a specific range of the present invention. The reason for the high water repellency is considered to be that the structural unit represented by Formula (2), which is a lipophilic component, is likely to be oriented outward when formed into a coating film by the configuration of the present invention. Favorable dispersibility in water is considered to be because the structural unit represented by Formula (1) is oriented outward in water to form a micelle, and is dispersed in water. Considering that the effects of the present invention are not achieved in the case of a binary polymer of the structural unit represented by Formula (1) and the structural unit represented by Formula (2) without the structural unit represented by Formula (3) (see, for example, Comparative Synthesis Examples 1 to 5 described below), it is considered that the structural unit represented by Formula (3) plays an important role in the control of the orientation as described above. The estimation mechanism that achieves the effects of the present invention does not limit the technical scope of the present invention at all.

Hereinafter, the copolymer of the present invention will be described.

### [Structural unit represented by Formula (1)]

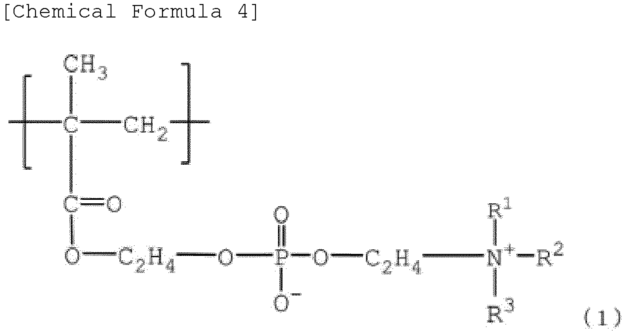

In the formula, R¹, R², and R³ each independently represent a substituted or unsubstituted linear or branched alkyl group having 1 to 30 carbon atoms.

Examples of the alkyl group having 1 to 30 carbon atoms include a methyl group, an ethyl group, a n-propyl group, an isopropyl group, a 2-methylpropyl group, a n-butyl group, an isobutyl group, a sec-butyl group, a tert-butyl group, a n-pentyl group, an iso-amyl group, a tert-pentyl group, a neopentyl group, a n-hexyl group, a 3-methylpentane-2-yl group, a 3-methylpentane-3-yl group, a 4-methylpentyl group, a 4-methylpentane-2-yl group, a 1,3-dimethylbutyl group, a 3,3-dimethylbutyl group, a 3,3-dimethylbutane-2-yl group, a n-heptyl group, a 1-methylhexyl group, a 3-methylhexyl group, a 4-methylhexyl group, a 5-methylhexyl group, a 1-ethylpentyl group, a 1-(n-propyl)butyl group, a 1,1-dimethylpentyl group, a 1,4-dimethylpentyl group, a 1,1-diethylpropyl group, a 1,3,3-trimethylbutyl group, a 1-ethyl-2,2-dimethylpropyl group, a n-octyl group, a 2-ethylhexyl group, a 2-methylhexane-2-yl group, a 2,4-dimethylpentane-3-yl group, a 1,1-dimethylpentane-1-yl group, a 2,2-dimethylhexane-3-yl group, a 2,3-dimethylhexane-2-yl group, a 2,5-dimethylhexane-2-yl group, a 2,5-dimethylhexane-3-yl group, a 3,4-dimethylhexane-3-yl group, a 3,5-dimethylhexane-3-yl group, a 1-methylheptyl group, a 2-methylheptyl group, a 5-methylheptyl group, a 2-methylheptane-2-yl group, a 3-methylheptane-3-yl group, a 4-methylheptane-3-yl group, a 4-methylheptane-4-yl group, a 1-ethylhexyl group, a 2-ethylhexyl group, a 1-propylpentyl group, a 2-propylpentyl group, a 1,1-dimethylhexyl group, a 1,4-dimethylhexyl group, a 1,5-dimethylhexyl group, a 1-ethyl-1-methylpentyl group, a 1-ethyl-4-methylpentyl group, a 1,1,4-trimethylpentyl group, a 2,4,4-trimethylpentyl group, a 1-isopropyl-1,2-dimethylpropyl group, a 1,1,3,3-tetramethylbutyl group, a n-nonyl group, a 1-methyloctyl group, a 6-methyloctyl group, a 1-ethylheptyl group, a 1-(n-butyl)pentyl group, a 4-methyl-1-(n-propyl)pentyl group, a 1,5,5-trimethylhexyl group, a 1,1,5-trimethylhexyl group, a 2-methyloctane-3-yl group, a n-decyl group, a 1-methylnonyl group, a 1-ethyloctyl group, a 1-(n-butyl)hexyl group, a 1,1-dimethyloctyl group, a 3,7-dimethyloctyl group, a n-undecyl group, a 1-methyldecyl group, a 1-ethylnonyl group, a n-dodecyl group, a 1-methylundecyl group, a n-tridecyl group, a n-tetradecyl group, a 1-methyltridecyl group, a n-pentadecyl group, a n-hexadecyl group, a n-heptadecyl group, a n-octadecyl group, a n-nonadecyl group, a n-eicosyl group, and the like.

Among them, in consideration of stability in an aqueous system, the alkyl group is preferably an alkyl group having 1 to 8 carbon atoms, more preferably an alkyl group having 1 to 3 carbon atoms, and particularly preferably a methyl group. That is, in a preferred embodiment, R¹ to R³ are each independently a linear or branched alkyl group having 1 to 3 carbon atoms which may be substituted, more preferably a methyl group which may be substituted, and particularly preferably a (unsubstituted) methyl group.

Examples of the substituent in the substituted linear or branched alkyl group having 1 to 30 carbon atoms include halogen atoms (F, Cl, Br, and I), an unsubstituted linear or branched alkoxy group having 1 to 4 carbon atoms, an unsubstituted linear or branched alkenyl group having 2 to 4 carbon atoms, an unsubstituted cycloalkyl group having 3 to 6 carbon atoms, a hydroxyl group, an amino group, and the like. The alkyl group having 1 to 30 carbon atoms is preferably unsubstituted in consideration of stability in an aqueous system. The substituent does not include an alkyl group.

When R¹ to R³ are a methyl group, the structural unit represented by Formula (1) is a structural unit derived from 2-methacryloyloxyethyl phosphorylcholine (MPC) represented by the following formula.

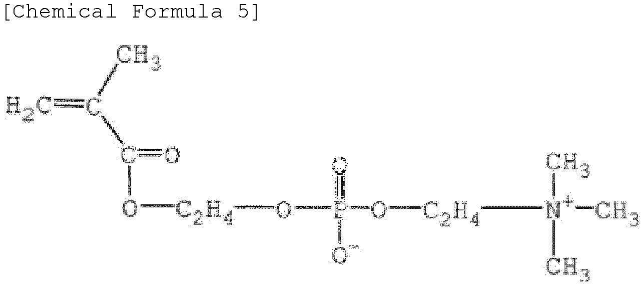

The content of the structural unit represented by Formula (1) is more than 20 mass% and less than 50 mass% in the copolymer. When the content of the structural unit represented by Formula (1) is 20 mass% or less, dispersibility in water is significantly deteriorated. When the content of the structural unit represented by Formula (1) is 50 mass% or more, the water repellency of a coating film is significantly deteriorated. The content of the structural unit represented by Formula (1) in the copolymer is preferably 25 to 45 mass%, more preferably 25 to 40 mass%, and still more preferably more than 25 mass% and less than 40 mass%.

### [Structural unit represented by Formula (2)]

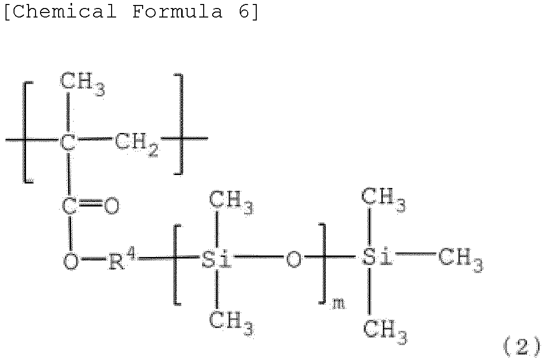

In Formula (2), R⁴ represents an alkylene group having 2 to 10 carbon atoms which may be substituted. R⁴ is preferably a linear or branched alkylene group having 2 to 8 carbon atoms, more preferably 2 to 6 carbon atoms, still more preferably an alkylene group having 2 to 4 carbon atoms, particularly preferably an alkylene group having 2 to 3 carbon atoms, and most preferably a trimethylene group (-CH₂CH₂CH₂-). Examples of the alkylene group having 2 to 10 carbon atoms include an ethylene group, a propylene group, a trimethylene group, a tetramethylene group, a propylene group, a butylene group, an ethylethylene group, a pentamethylene group, a hexamethylene group, a heptamethylene group, an octamethylene group, and the like.

Examples of the substituent optionally present in the alkylene group having 2 to 10 carbon atoms include halogen atoms (F, Cl, Br, and I), an unsubstituted linear or branched alkoxy group having 1 to 4 carbon atoms, an unsubstituted linear or branched alkenyl group having 2 to 4 carbon atoms, an unsubstituted cycloalkyl group having 3 to 6 carbon atoms, a hydroxyl group, an amino group, and the like. The alkylene group having 2 to 10 carbon atoms is preferably unsubstituted in consideration of water repellency of a coating film. The substituent does not include an alkyl group.

In Formula (2), m represents an integer of 1 to 30. When m is an integer of 31 or more, dispersibility in water is deteriorated. m is preferably an integer of 1 to 24, and is, for example, an integer of 1 to 20, an integer of 1 to 18, an integer of 1 to 16, an integer of 1 to 12, an integer of 6 to 24, an integer of 6 to 20, an integer of 6 to 18, an integer of 6 to 16, an integer of 6 to 12, an integer of 8 to 24, an integer of 8 to 20, an integer of 8 to 16, an integer of 8 to 12, 8, 9, 10, 11, and 12. In a preferred embodiment, m is 11.

The content of the structural unit represented by Formula (2) is more than 0 mass% and less than 40 mass% in the copolymer. When the content of the structural unit represented by Formula (1) is 40 mass% or more, dispersibility in water is significantly deteriorated. When the structural unit represented by Formula (2) is 0 mass%, the water repellency of a coating film is significantly deteriorated. The content of the structural unit represented by Formula (2) in the copolymer is preferably 5 to 35 mass%, more preferably 10 to 30 mass%, and particularly preferably more than 10 mass% and less than 30 mass%.

### [Structural unit represented by Formula (3)]

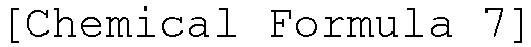

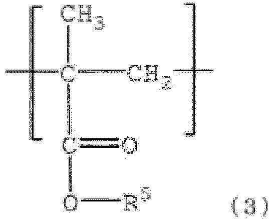

In the formula, R⁵ represents a linear or branched alkyl group having 1 to 30 carbon atoms which may be substituted. When the number of carbon atoms of R⁵ is 31 or more, dispersibility in water is deteriorated.

The alkyl group having 1 to 30 carbon atoms is the same as that described in the column of the above Formula (1). In particular, in consideration of solubility in water, the alkyl group is preferably an alkyl group having 1 to 8 carbon atoms. That is, in a preferred embodiment, R⁵ is a linear or branched alkyl group having 1 to 8 carbon atoms which may be substituted, more preferably a linear or branched alkyl group having 1 to 6 carbon atoms which may be substituted, still more preferably a linear or branched alkyl group having 1 to 4 carbon atoms which may be substituted, particularly preferably a n-butyl group may be substituted, and most preferably a (unsubstituted) n-butyl group.

Examples of the substituent in the substituted linear or branched alkyl group having 1 to 30 carbon atoms include halogen atoms (F, Cl, Br, and I), an unsubstituted linear or branched alkoxy group having 1 to 4 carbon atoms, an unsubstituted linear or branched alkenyl group having 2 to 4 carbon atoms, an unsubstituted cycloalkyl group having 3 to 6 carbon atoms, a hydroxyl group, an amino group, and the like. The alkyl group having 1 to 30 carbon atoms is preferably unsubstituted in consideration of both stability in an aqueous system and makeup retention. The substituent does not include an alkyl group.

The content of the structural unit represented by Formula (3) in the copolymer is preferably 20 to 70 mass%, more preferably 30 mass% or more and less than 70 mass%, still more preferably 35 to 65 mass%, and particularly preferably 40 to 60 mass%. When the content of the structural unit represented by Formula (3) is in the range, it is further easy to achieve both water resistance of a coating film to be formed and dispersibility in water.

### [Other structural units]

The copolymer of the present invention may contain structural units (hereinafter, also simply referred to as other structural units) other than the structural units represented by Formulas (1) to (3). However, in the present invention, from the viewpoint of achieving both water resistance of a coating film to be formed and dispersibility in water, the content of the other structural units in the copolymer is preferably less than 1 mass% and more preferably less than 0.5 mass%, and it is preferable that the other structural units are not substantially contained. The expression "not substantially contained" means that the content of the other structural units is preferably 0.01 mass% or less (lower limit: 0 mass%), more preferably 0.001 mass% or less (lower limit: 0 mass%), and particularly preferably 0 mass . A preferred embodiment of the present invention is a copolymer composed of the structural units represented by Formulas (1) to (3).

### [Weight average molecular weight]

The weight average molecular weight (Mw) of the copolymer of the present invention is not particularly limited as long as the object of the present invention can be achieved, but is preferably 20,000 or more and more preferably 30,000 or more. When the weight average molecular weight is the above lower limit or more, the water repellency of a coating film is further improved. The weight average molecular weight (Mw) of the copolymer of the present invention is not particularly limited as long as the object of the present invention can be achieved, but is preferably 100,000 or less, more preferably 80,000 or less, still more preferably 70,000 or less, and particularly preferably 60,000 or less. When the weight average molecular weight is the above upper limit or less, solubility in an aqueous system is further improved, and stability is further improved. The weight average molecular weight (Mw) of the copolymer of the present invention is, for example, 20,000 to 100,000, and further, is, for example, 20,000 to 80,000, 20,000 to 70,000, 30,000 to 70,000, and 30,000 to 60,000. The weight average molecular weight of the copolymer of the present invention is calculated by a multi-angle light scattering method (MALS method) using a multi-angle light scattering detector.

### [Production method]

The copolymer of the present invention containing the structural units represented by Formulas (1) to (3) as repeating units can be obtained by polymerization by a known polymerization method in the presence of a solvent. Although not particularly limited, a monomer composition containing a monomer for forming each structural unit may be polymerized in the presence of a radical polymerization initiator. Examples of the radical polymerization initiator include organic peroxides such as t-butyl peroxy neodecanoate, benzoyl peroxide, and lauroyl peroxide; azo-based compounds such as α,α'-azobisisobutyronitrile, 2,2'-azobis(2,4-dimethylvaleronitrile), and 2,2'-azobis(2-methylbutyronitrile); persulfate-based polymerization initiators such as potassium persulfate and ammonium persulfate; and the like. These polymerization initiators can be used singly or in combination of two or more kinds thereof. The amount of the polymerization initiator used is usually 0.01 to 10 parts by mass and preferably 0.1 to 5 parts by mass with respect to 100 parts by mass of the total monomers.

Examples of the monomer for forming the structural unit of Formula (1) include monomers represented by the following formula. 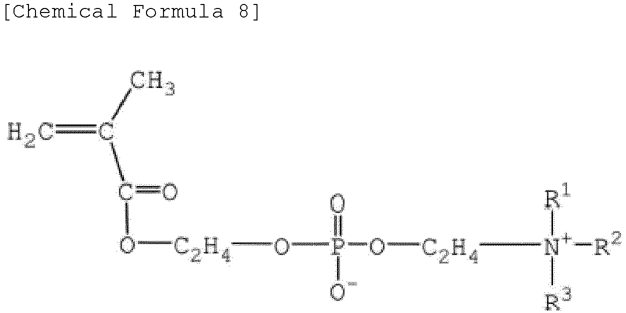 in the above formula, R¹ to R³ have the same meaning as R¹ to R³ in Formula (1).

Specific examples of the monomer for forming the structural unit of Formula (1) include 2-methacryloyloxyethyl phosphorylcholine (MPC) represented by the following formula.

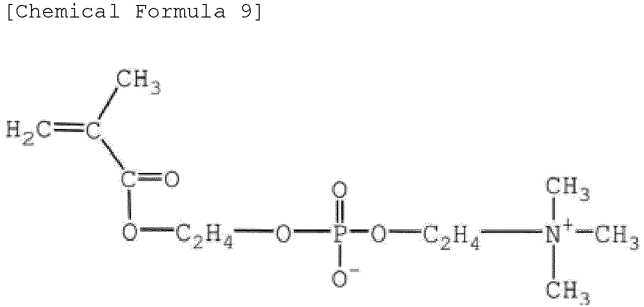

Examples of the monomer for forming the structural unit of Formula (2) include monomers represented by the following formula.

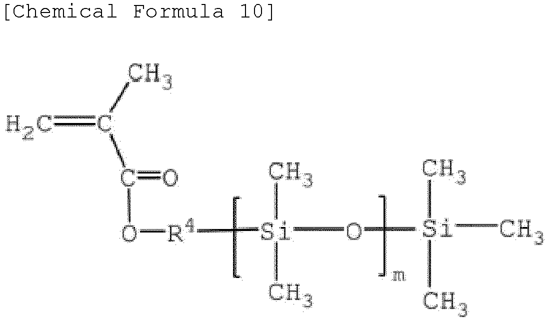

In the above formula, R⁴ has the same meaning as R⁴ in Formula (2). In the above formula, m has the same meaning as m in Formula (2). The monomer represented by the above formula may be abbreviated as "SiMA".

Examples of the monomer for forming the structural unit of Formula (3) include alkyl methacrylate. Specific examples thereof include methyl methacrylate, ethyl methacrylate, propyl methacrylate, butyl methacrylate, and 2-ethylhexyl methacrylate, and the like.

The content of each structural unit coincides with the addition amount of each monomer in the production stage. The content of each structural unit can be grasped by measurement by NMR, molecular weight measurement, or the like.

The polymerization may be performed in an inert gas atmosphere such as nitrogen. For the polymerization, a solution polymerization method, a suspension polymerization method, a bulk polymerization method, a precipitation polymerization method, or the like can be used. Among them, a solution polymerization method is particularly preferable because it is easy to adjust the molecular weight of the copolymer to be obtained to an optimum range. Also, the purification of the copolymer of the present invention can be conducted by a known purification method such as a reprecipitation method or a dialysis method. The copolymer of the present invention may be either a random copolymer or a block copolymer, but for example, the copolymer of the present invention is a random copolymer.

Examples of the solvent used in the polymerization of the copolymer of the present invention include aromatic hydrocarbons such as benzene, toluene, and xylene; ketones such as methyl ethyl ketone and methyl isobutyl ketone; esters such as ethyl acetate and butyl acetate; alcohols such as isopropanol, ethanol, and methanol; and water, and one or a combination of two or more kinds thereof can be used. The copolymer can also be polymerized in a paraffinic solvent such as light isoparaffin, isododecane, or isohexadecane.

The polymerization reaction temperature of the copolymer of the present invention is not particularly limited as long as it is in a temperature range in which a normal radical polymerization initiator can be used, but the polymerization reaction is usually carried out in a range of 40 to 120°C. The reaction temperature varies depending on the radical polymerization initiator to be used, the type of monomer, and the reaction temperature, but the polymerization reaction is usually carried out for 2 to 24 hours. When the polymerization time is too short, the amount of residual monomers is large and the yield is low, which is not preferable.

### [Cosmetic]

The copolymer of the present invention (hereinafter, also simply referred to as the copolymer) itself can be used as a raw material for producing various cosmetics. That is, the present invention also provides a cosmetic containing a copolymer. Since the cosmetic preparation contains a copolymer, water resistance to sweat and the like is enhanced, and makeup retention is improved. Since the cosmetic contains a copolymer, secondary adhesion of the cosmetic to a mask or the like can be suppressed. Since the cosmetic contains a copolymer, stability over time is high. Details of exerting the above effect by using the copolymer of the present invention are unknown, but it is estimated as follows.

The cosmetic of the present invention has high water resistance of a cosmetic film formed by containing a copolymer having high water repellency. Since the cosmetic of the present invention contains a copolymer, sustainability of a cosmetic effect is high (makeup retention is favorable). The reason for this is considered that by containing a copolymer having high water repellency, the cosmetic film has high water resistance as described above, and in addition thereto, the structural unit represented by Formula (1) has a structure similar to that of a phospholipid which is an intercellular lipid, and has a specific characteristic of retaining bound water, and further, the copolymer contains the structural units of Formulas (1) to (3) at a blending ratio of a specific amount, so that the polymer conformation is improved, and the structural unit represented by Formula (1) is likely to be oriented toward the skin side to improve the skin affinity of the cosmetic. The cosmetic of the present invention can suppress secondary adhesion of the cosmetic to a mask or the like. The reason for this is considered that in the copolymer of the present invention, since the structural unit represented by Formula (1) has a specific characteristic of retaining bound water and the copolymer contains the structural units of Formulas (1) to (3) at a blending ratio of a specific amount, the polymer conformation is improved, and the structural unit of Formula (2) having low friction and low tackiness (stickiness) is likely to be arranged on the outermost surface of a coating film. Since the copolymer has favorable dispersibility in water, it is considered that the copolymer has high stability over time when blended in a cosmetic.

Since the cosmetic of the present invention contains a copolymer, the cosmetic also has a feeling such as no burden at the time of application and no burden over time.

The content of the copolymer in the cosmetic is appropriately set according to a desired feel and function, and is, for example, 0.01 to 20 mass%, more preferably 0.1 to 15 mass%, still more preferably 0.1 to 10 mass%, particularly preferably more than 0.5 mass% and less than 8 mass%, and particularly preferably 1 to 5 mass%.

Examples of the cosmetic include basic cosmetics such as a lotion, an emulsion, a cream, a serum, a massage cosmetic, a pack cosmetic, a hand cream, a body lotion, a body cream, an eye cream, and a sunscreen; makeup cosmetics such as foundation and makeup base; and hair cosmetics such as hair cream and hair wax. Examples of the property of the cosmetic include a liquid, a gel, an emulsion, a cream, a semi-solid, and a solid, and the cosmetic preparation is preferably a liquid since a large amount of an aqueous component can be blended.

Since the copolymer of the present invention has favorable dispersibility in water, the copolymer can be stably blended in an aqueous system. The stability is maintained even after a lapse of a long time. For this reason, as a dosage form of the cosmetic, a water-based(including a solubilizing type and a powder-containing bilayer type) or an emulsified cosmetic (such as oil-in-water type or water-in-oil type), which is a dosage form blended with water, is preferable, and a water-based or oil-in-water emulsified cosmetic is more preferable since the aqueous phase is a foreign phase and contributes to the stability of the entire system.

Since the property that the dispersibility of the copolymer of the present invention in water is favorable is effectively exhibited, the cosmetic preferably contains water. The water is not particularly limited as long as it is generally used for a cosmetic, and examples thereof include normal water, purified water, ion-exchanged water, hot spring water, deep water, distilled water with steam derived from plants such as lavender water, rose water, and orange flower water, and the like. These may be used singly or in combination of two or more kinds thereof. The content of water in the cosmetic is not particularly limited, but is preferably 20 to 99 mass%, more preferably 30 to 95 mass%, and still more preferably 40 to 95 mass%.

Various components usually used in the cosmetic can be blended in the cosmetic. As the components usually blended in the cosmetic, an oil, surfactants (an anionic surfactant, a nonionic surfactant, an amphoteric surfactant, and a cationic surfactant), thickeners (a gelling agent and a polymer), a coloring agent, a powder other than the coloring agent, a lower alcohol such as ethanol, a polyhydric alcohol, an ultraviolet absorber, a pH adjusting agent, an antioxidant, a metal chelating agent, an antiseptic agent, a fragrance, various agents, and the like can be arbitrarily blended.

Examples of the an oil agent include hydrocarbons such as isododecane, isohexadecane, light isoparaffin, liquid paraffin, squalane, squalene, α-olefin oligomer, polybutene, liquid isoparaffin, heavy liquid isoparaffin, polyisobutylene, and hydrogenated polyisobutene, animal and vegetable oils such as oilseed rape oil, avocado oil, almond oil, apricot kernel oil, perilla oil, orange oil, olive oil, kiwi seed oil, sesame oil, wheat germ oil, rice germ oil, rice bran oil, safflower oil, sage oil, soybean oil, camellia sinensis seed oil, corn oil, rapeseed oil, evening primrose oil, camellia oil, persic oil, Coix lacryma-jobi seed oil, peanut oil, sunflower oil, grape seed oil, meadowfoam oil, rosemary oil, jojoba oil, macadamia nut oil, lavender oil, rosehip oil, and mink oil; esters such as glyceryl tri 2-ethylhexanoate, isotridecyl isononanoate, isononyl isonononanoate, cetyl 2-ethylhexanoate, isopropyl myristate, isopropyl palmitate, 2-ethylhexyl palmitate, octyldodecyl myristate, glyceryl trioctanoate, glyceryl tri(capryl caprate), glyceryl stearate, glyceryl diisostearate, glyceryl triisostearate, decaglyceryl decaisostearate (polyglyceryl decaisostearate-10), propylene glycol dicaprate, neopentyl glycol dicaprate, polyglyceryl triisostearate, diisostearyl malate, neopentyl glycol diethylhexanoate, polyglyceryl decastearate-10, pentaerythrityl tetraisostearate, pentaerythritol tetra 2-ethylhexanoate, dipentaerythritol pentaisostearate, dialkyl carbonate, tritritridecyl trimellitate, bisethoxy diglycol cyclohexane-1,4-dicarboxylic acid, and dimer dilinoleyl hydrogenated rosin condensate; fatty acids such as oleic acid, isostearic acid, and stearic acid; higher alcohols such as oleyl alcohol, 2-octyldodecanol, 2-decyltetradecanol, isostearyl alcohol, 2-hexyldecanol, and cetanol; silicone oils such as dimethylpolysiloxane (dimethicone), methyltrimethicone, methylphenylpolysiloxane, octamethylcyclotetrasiloxane, decamethylcyclopentasiloxane, tetramethyltetrahydrogencyclotetrasiloxane, tetramethyltetraphenylcyclotetrasiloxane, tetramethyltetratrifluoropropylcyclotetrasiloxane, pentamethylpentatrifluoropropylcyclopentasiloxane, polyether-modified methylpolysiloxane, oleyl-modified methylpolysiloxane, and polyvinylpyrrolidone-modified methylpolysiloxane; fluorine-based oils such as perfluoropolyether, perfluorodecane, and perfluorooctane; lanolin derivatives such as lanolin acetate, isopropyl lanolin fatty acid, and lanolin alcohol; liquid oils of ultraviolet absorber or the like such as 2-ethylhexyl p-methoxycinnamate and ethylhexyl salicylate; paste oils such as cocoa butter, shea butter, castor oil, hydrogenated castor oil, hydrogenated coconut oil, petrolatum, monostearic acid hydrogenated castor oil, monohydroxystearic acid hydrogenated castor oil, cholesteryl hydroxystearate, dipentaerythritol fatty acid ester, N-lauroyl-L-glutamate-di(octyldodecyl/cholesteryl/behenyl), N-lauroyl-L-glutamate-di(octyldodecyl/phytosteryl/behenyl), macadamia nut oil fatty acid phytosteryl, and dimer dilinoleic acid (phytosteryl/isostearyl/cetyl/stearyl/behenyl); solid oils such as paraffin wax, ceresin wax, microcrystalline wax, polyethylene wax, Fischer-Tropsch wax, stearic acid, behenic acid, cetyl palmitate, polyethylene glycol distearate, glyceryl tribehenate, cholesterol, phytosterol, stearyl-modified polysiloxane, hardened oil, petrolatum, and palm oil. These oil agents may be used singly or in combination of two or more kinds thereof.

Examples of the anionic surfactant include fatty acid soaps, acyl glutamates, alkyl phosphates, polyoxyalkylene-added alkyl phosphates such as polyoxyethylene lauryl ether phosphate, polyoxyethylene cetyl ether phosphate, and polyoxyethylene stearyl ether phosphate, and the like. These anionic surfactants may be used singly or in combination of two or more kinds thereof.

Examples of the nonionic surfactant include sorbitan fatty acid ester, glycerin fatty acid ester, polyglycerin fatty acid ester, propylene glycol fatty acid ester, polyethylene glycol fatty acid ester, sucrose fatty acid ester, polyoxyethylene alkyl ether, polyoxypropylene alkyl ether, polyoxyethylene alkyl phenyl ether, polyoxyethylene fatty acid ester, polyoxyethylene sorbitan fatty acid ester, polyoxyethylene sorbitol fatty acid ester, polyoxyethylene glycerin fatty acid ester, polyoxyethylene propylene glycol fatty acid ester, polyoxyethylene castor oil, polyoxyethylene hydrogenated castor oil, polyoxyethylene hydrogenated castor oil fatty acid ester, polyoxyethylene phytostanol ether, polyoxyethylene phytosterol ether, polyoxyethylene cholesterol ether, polyoxyethylene cholesteryl ether, polyoxyalkylene-modified organopolysiloxane, polyoxyalkylene-alkyl-co-modified organopolysiloxane, alkanol amide, sugar ether, sugar amide, and the like. These nonionic surfactants may be used singly or in combination of two or more kinds thereof.

Examples of the amphoteric surfactant include imidazoline-based amphoteric surfactants (for example, sodium 2-undecyl-N,N,N-(hydroxyethylcarboxymethyl)-2-imidazoline, 2-cocoyl-2-imidazoliniumhydroxide-1-carboxyethyloxydisodium salt, and the like); betaine-based surfactants (for example, 2-heptadecyl-N-carboxymethyl-N-hydroxyethylimidazolinium betaine, lauryldimethylaminoacetic acid betaine, alkyl betaine, amide betaine, sulfobetaine, and the like); phospholipids (for example, lecithin, hydrogenated lecithin, and the like); and the like. Examples of the origin of lecithin include soybean, egg, and the like.

A coloring agent is not particularly limited to a shape such as a spherical shape, a plate shape, a spindle shape, or a needle shape, a particle size such as fumy particles, fine particles, and a pigment level, a particle structure such as a porous or non-porous structure, or the like as long as it is a coloring agent generally used for cosmetics, and an inorganic pigment, an organic pigment, a bright pigment, a metal, and the like can be used. Specific examples of the powder include white inorganic pigments such as titanium oxide, zinc oxide, cerium oxide, and barium sulfate; colored inorganic pigments such as iron oxide, carbon black, chromium oxide, chromium hydroxide, Prussian blue, ultramarine, and colcothar; bright pigments such as titanium mica, bismuth oxychloride, organic pigment-treated titanium mica, titanium dioxide-coated mica, titanium dioxide-coated synthetic phlogopite, titanium dioxide-coated bismuth oxychloride, iron oxide mica titanium, Prussian blue-treated mica titanium, carmine-treated mica titanium, a fish scale, titanium dioxide-coated glass powder, resin laminate powder including polyethylene terephthalate-aluminum-epoxy laminate powder, polyethylene terephthalate-polyolefin laminate film powder, and polyethylene terephthalate-polymethylmethacrylate laminate film powder; organic pigment powders such as Red No. 201, Red No. 202, Red No. 205, Red No. 226, Red No. 228, Orange No. 203, Orange No. 204, Blue No. 404, and Yellow No. 401; organic pigment powder of zirconium, barium, or aluminum lake such as Red No. 3, Red No. 104, Red No. 106, Orange No. 205, Yellow No. 4, Yellow No. 5, Green No. 3, and Blue No. 1; metal powders such as aluminum powder, gold powder, and silver powder; composite powder such as microparticulate titanium oxide-coated mica titanium, microparticulate zinc oxide-coated titanium mica, barium sulfate-coated titanium mica, titanium oxide-containing silicon dioxide, and zinc oxide-containing silicon dioxide; and the like, examples of a natural dye include carminic acid, laccaic acid, carthamin, brazilin, crocin, and the like, and one or two or more kinds thereof can be used as necessary. These may be subjected to surface treatment using one or two or more of a fluorine-based compound, a silicone-based compound, a metal soap, lecithin, hydrogenated lecithin, collagen, hydrocarbon, higher fatty acid, higher alcohol, ester, wax, a surfactant, and the like.

The powder other than the coloring agent is not particularly limited to a shape such as a plate shape, a spindle shape, or a needle shape, a particle size such as fumy particles or fine particle level, a particle structure such as a porous or non-porous structure, or the like as long as it is generally used as a raw material for the cosmetic. Examples of the powder other than the coloring agent include inorganic powders such as aluminum oxide, cerium oxide, magnesium oxide, zirconium oxide, magnesium carbonate, calcium carbonate, aluminum silicate, magnesium silicate, aluminum magnesium silicate, mica, synthetic mica, synthetic sericite, sericite, talc, kaolin, silica, silicon carbide, and boron nitride; and organic powders such as magnesium stearate, zinc stearate, N-acyllysine, nylon, polymethyl methacrylate, organopolysiloxane powder, and cellulose powder, and one or two or more kinds thereof can be used. These powders may be used singly or in combination of two or more kinds thereof, and may be subjected to surface treatment such as oil treatment, silicone compound treatment, or water-soluble polymer treatment.

Examples of the gelling agent include dextrin fatty acid esters such as octanoic acid dextrin, lauric acid dextrin, myristic acid dextrin, palmitic acid dextrin, palmitic acid/2-ethylhexanoic acid dextrin, stearic acid dextrin, isostearic acid dextrin, palmitic acid/stearic acid dextrin, oleic acid dextrin, palmitic acid/hexyldecanoic acid dextrin, behenic acid dextrin, and coconut oil fatty acid dextrin; sucrose fatty acid esters such as sucrose stearate ester and sucrose palmitate ester; fructo-oligosaccharide fatty acid esters such as fructo-oligosaccharide stearic acid ester; inulin fatty acid esters such as inulin stearic acid ester; organically modified bentonites such as dimethyldistearylammonium hectorite, dimethyldistearylammonium bentonite, benzyldimethyldistearylammonium hectorite, disteardimonium hectorite, dioctadecyldimethylammonium salt-modified montmorillonite, octadecyldimethylbenzylammonium salt-modified montmorillonite, and dihexadecyldimethylammonium salt-modified montmorillonite; glycerin oligoesters such as glycerin-based oligoester-based gelling agent synthesized from glycerin, eicosanedioic acid and behenic acid (referred to as (behenic acid/eicosanedioic acid)glyceryl), glyceryl 2-ethyloctadecanedioate laurate, glyceryl tetradecanedioate myristate, glyceryl palmitate eicosanedioate, glyceryl stearate hexadecanedioate, glyceryl isostearate eicosanedioate, glyceryl octadecanedioate behenate, glyceryl 2-ethylhexanoate dodecanedioate, and glyceryl 2-ethyloctadecanedioate oleate; fumed silicic anhydride; metal soaps such as aluminum isostearate, zinc stearate, and magnesium stearate; and the like. These gelling agents may be used singly or in combination of two or more kinds thereof.

Examples of the polymer include water-soluble polymers such as carrageenan, xanthan gum, methyl cellulose, hydroxyethyl cellulose, carboxymethyl cellulose, a carboxyvinyl polymer, an alkyl-modified carboxyvinyl polymer, polyvinyl alcohol, polyvinyl pyrrolidone, a (Na acrylate/Na acryloyldimethyltaurine) copolymer, and a (PEG-240/decyltetradecase-20/HDI) copolymer; terpene-based resin, silicone resin (for example, graft copolymer of acrylic polymer and dimethylpolysiloxane (acrylates/dimethicone) copolymer (INCI name)), a (meth)acrylic acid-(meth)acrylic acid alkyl ester copolymer (acrylates copolymer (INCI name)), trimethylsiloxysilicic acid, polymethylsilsesquioxane, hydrocarbon resin, vinyl acetate-based resin (for example, polyvinyl acetate), a rosin-based resin (for example, pentaerythrityl rosinate, pentaerythrityl hydrogenated rosinate, and glyceryl hydrogenated abietate), acrylic resin (for example, polyethyl acrylate, polybutyl methacrylate, an acrylic acid/alkyl acrylate copolymer, and an alkyl acrylate/vinyl acetate copolymer); and the like. Even when the copolymer of the present application and the water-soluble polymer are combined, there is no viscosity reduction or precipitation due to a polymer complex, and this event has been confirmed also in the following Examples. In this respect, the cosmetic may further contain a water-soluble polymer.

Examples of the polyhydric alcohol include propylene glycol, 1,3-butylene glycol, dipropylene glycol, tripropylene glycol, and the like. These polymers may be used singly or in combination of two or more kinds thereof.

Examples of the ultraviolet absorber include cinnamic acid derivatives, aminobenzoic acid derivatives, salicylic acid derivatives, benzophenone derivatives, phenylbenzimidazole derivatives, phenylbenzotriazole derivatives, silicone derivatives, and the like. Specific examples thereof include 2-ethylhexyl p-methoxycinnamate, octocrylene, polysilicone-15, t-butylmethoxydibenzoylmethane, ethylhexyltriazone, diethylaminohydroxybenzoyl hexyl benzoate, bisethylhexyloxyphenol methoxyphenyltriazine, oxybenzone, methylene bisbenzotriazolyl tetramethylbutylphenol, phenylbenzimidazole sulfonic acid, homosalate, ethyl hexyl salicylate, and the like. The ultraviolet absorber may be included in the oil.

As a method for producing a cosmetic, a cosmetic can be obtained by dissolving the copolymer of the present invention in an aqueous system and performing emulsification or the like as necessary.

### Examples

The effects of the present invention will be described with the following Examples and Comparative Examples. In Examples, the expression "part(s)" or "%" may be used, but unless otherwise specified, the expression represents "part(s) by mass" or "mass%". Unless otherwise specified, each operation is performed at room temperature (25°C).

### [Synthesis Example 1]

In a four-necked flask, 30.0 g of 2-methacryloyloxyethyl phosphorylcholine (MPC, manufactured by NOF CORPORATION), 10.0 g of silicone methacrylate (SiMA, manufactured by Shin-Etsu Chemical Co., Ltd., R⁴ is a trimethylene group and m is 11 in Formula (2)), and 60.0 g of butyl methacrylate (Blemmer BMA, manufactured by NOF CORPORATION) were put, dissolved in 233.3 g of ethanol, and blown with nitrogen gas for 30 minutes. Thereafter, 2.0 g of t-butyl peroxy neodecanoate (PERBUTYL (registered trademark in Japan) ND (PB-ND), manufactured by NOF CORPORATION) was added as a polymerization initiator, and a polymerization reaction was performed at 70°C for 6 hours. After the polymerization reaction, volatile components were distilled off under reduced pressure to obtain a polymer powder. The molecular weight of the polymer was analyzed by the MALS method. A multi-angle light scattering detector (DAWN HELEOS II 8+) was used as an apparatus, and isopropanol was used as a medium. The weight average molecular weight thereof was 34,000.

### [Synthesis Examples 2 to 4]

A polymer was produced according to the same procedure as in Synthesis Example 1, except that the types and amounts of monomers shown in the following Table 1 were used.

### [Synthesis Comparative Examples 1 to 12]

A polymer was produced according to the same procedure as in Synthesis Example 1, except that the types and amounts of monomers shown in the following Table 1 were used.

### (Evaluation method 1: dispersibility in water)

With 90 g of water, 10 g of the copolymer produced in Synthesis Example or Synthesis Comparative Example was mixed, the mixture was heated to 70° for 2 hours and then cooled at normal temperature, and whether or not stable dispersion was possible was visually confirmed. The results are also shown together in Table 1 below.
∘: The copolymer was dissolved in a transparent manner.
×: Cloudiness was observed or the polymer precipitated in the lower layer.

### (Evaluation method 2: contact angle of coating film)

A 10 mass% aqueous solution of the copolymer produced in Synthesis Example or Synthesis Comparative Example was prepared, and a film was formed with a 25 um doctor blade. The static contact angle of water was measured by a droplet method. When the evaluation is 2 or more, it can be said that favorable water repellency is achieved. The results are also shown together in Table 1 below.
5: 100° or more
4: 80° or more and less than 100°
3: 60° or more and less than 80°
2: 50° or more and less than 60°
1: Less than 50°

**[Table 1]**

| | MPC | SiMA | BMA | Property | Dispersibility in water | Water repellency of coating film |
|---|---|---|---|---|---|---|
| Synthesis Example 1 | 30 | 10 | 60 | White powder | ○ | 4 |
| Synthesis Example 2 | 30 | 20 | 50 | White powder | ○ | 5 |
| Synthesis Example 3 | 30 | 30 | 40 | White powder | ○ | 4 |
| Synthesis Example 4 | 40 | 10 | 50 | White powder | ○ | 3 |
| Synthesis Comparative Example 1 | 90 | 10 | 0 | White powder | ○ | 1 |
| Synthesis Comparative Example 2 | 80 | 20 | 0 | White powder | ○ | 1 |
| Synthesis Comparative Example 3 | 70 | 30 | 0 | White rubber | × | - |
| Synthesis Comparative Example 4 | 50 | 50 | 0 | White rubber | × | - |
| Synthesis Comparative Example 5 | 30 | 70 | 0 | White rubber | × | - |
| Synthesis Comparative Example 6 | 90 | 5 | 5 | White powder | ○ | 1 |
| Synthesis Comparative Example 7 | 80 | 10 | 10 | White powder | ○ | 1 |
| Synthesis Comparative Example 8 | 70 | 10 | 20 | White powder | ○ | 1 |
| Synthesis Comparative Example 9 | 50 | 10 | 40 | White powder | ○ | 1 |
| Synthesis Comparative Example 10 | 20 | 10 | 70 | White powder | × | - |
| Synthesis Comparative Example 11 | 10 | 10 | 80 | White powder | × | - |
| Synthesis Comparative Example 12 | 30 | 40 | 30 | White powder | × | - |

| | | | | | | |
|---|---|---|---|---|---|---|
| (Note) Abbreviations in Table 1 have the following meanings. MPC: 2-methacryloyloxyethyl phosphorylcholine (manufactured by NOF CORPORATION) SiMA: silicone methacrylate (manufactured by Shin-Etsu Chemical Co., Ltd.) BMA: butyl methacrylate (Blemmer BMA, manufactured by NOF CORPORATION) | | | | | | |

The molecular weight (Mw) of Synthesis Example 1 was 34000, the molecular weight (Mw) of Synthesis Example 2 was 52000, the molecular weight (Mw) of Synthesis Example 3 was 52000, and the molecular weight (Mw) of Synthesis Example 4 was 55000.

From the above results, the copolymer of Synthesis Examples had favorable dispersibility in water and high water repellency of a coating film.

### [Examples 1 to 5 and Comparative Examples 1 to 5: oil-in-water liquid foundation]

An oil-in-water liquid foundation having the composition (mass% with respect to 100 mass% of the entire cosmetic; the same applies hereinafter) shown in Table 2 was prepared by the following production method, and evaluation and determination were performed for items of (a) water resistance of a cosmetic film, (b) secondary adhesion-preventing effect of a cosmetic film, (c) no makeup deterioration over time, (d) no burden at the time of application, (e) no burden over time, and (f) stability of polymer formulation by the following evaluation method and determination criteria, and the results are shown together in Table 2.

### [Production method]

(1) Components 1 to 3 and 8 to 14 were uniformly mixed and heated to 75°C.
(2) Components 7, 15 to 22, 24, and 25 were uniformly mixed and heated to 75°C.
(3) (1) was added to (2), emulsified, and cooled.
(4) Components 4 to 6, Component 23, and Components 26 to 30 were treated with a three-membered roller.
(5) (4) was added to (3) and dispersed with a disper.
(6) Components 31 and 32 were added to (5) and then defoamed to obtain an oil-in-water liquid foundation.

### [Evaluation method]

### (a) Water resistance of cosmetic film

Each liquid foundation (10 mg) was applied to a PMMA plate (5 × 5 cm), and then dried at room temperature for 30 minutes, the plate was stuck to a cylindrical plastic container, 100 g of water was put in the container, the container was shaken for 1 minute, the state of collapse and thinning of the cosmetic film was visually observed, and water resistance was evaluated according to the following criteria for determination.

### (Criteria for determination)

The cosmetic film does not fall off at all: ⊙
The cosmetic film hardly falls off: o
The cosmetic film falls off: △
The cosmetic film considerably falls off: ×

### (b) Secondary adhesion-preventing effect of cosmetic film

Each liquid foundation (10 mg) was applied to a black artificial leather (5 × 5 cm), and then dried at room temperature for 30 minutes, a nonwoven fabric (5 × 5 cm) and a metal plate (5 × 5 cm) were placed thereon, and pressed at a pressure of 50 MPa with a hydraulic press machine, and then the secondary adhesion preventing effect was evaluated according to the following criteria for determination from the degree of color transfer to the nonwoven fabric.

### (Criteria for determination)

No adhesion to nonwoven fabric: ⊙
Almost no adhesion to nonwoven fabric: o
Adhesion to nonwoven fabric: △
Substantial adhesion to nonwoven fabric: ×

(c) no makeup deterioration over time, (d) no burden (spreading of the cosmetic is poor and the skin is pulled by the applying action) at the time of application, (e) no burden (feeling of tight feel on skin or feeling of blockage due to a cosmetic film) over time

As for no makeup deterioration over time of each liquid foundation, each liquid foundation was applied to the entire face, changes over time immediately after application and after 6 hours were visually observed by 20 cosmetic evaluation professional panels, each panel evaluated the degree of collapse of a cosmetic film by five grades according to the following criteria, each grade was given a score, and the average score of the scores of all the panels was further determined according to the following criteria for determination. Also as for no burden at the time of application and no burden over time, sensory evaluation was performed at the time of application and after 6 hours, each panel evaluated by five grades according to the following criteria, each grade was given a score, and the average score of the scores of all the panels was further determined according to the following criteria for determination.

### (Evaluation result): (score)

Very good: 5 points
Good: 4 points
Normal: 3 points
Slightly poor: 2 points
Poor: 1 point
(Criteria for determination): determination
More than 4.0: ⊙
More than 3.0 and 4.0 or less: o
More than 2.0 and 3.0 or less: △
2.0 or less: ×

### (f) Stability of polymer formulation

Each liquid foundation was left to stand still and stored in a thermostatic bath at 5°C for 1 month and then returned to room temperature, and the presence or absence of insolubilization of the blended polymer was confirmed.

### (Criteria for determination): determination

Insolubilization of the polymer is not observed at all: ⊙
Insolubilization of the polymer is observed: ×

**[Table 2-1] (mass%)**

| No. | Component | Example 1 | Example 2 | Example 3 | Example 4 | Example 5 | Comparative Example 1 | Comparative Example 2 | Comparative Example 3 | Comparative Example 4 | Comparative Example 5 |
|---|---|---|---|---|---|---|---|---|---|---|---|
| 1 | Stearic acid | 1 | 1 | 1 | 1 | 1 | 1 | 1 | 1 | 1 | 1 |
| 2 | Glyceryl stearate | 0.5 | 0.5 | 0.5 | 0.5 | 0.5 | 0.5 | 0.5 | 0.5 | 0.5 | 0.5 |
| 3 | Cetanol | 0.5 | 0.5 | 0.5 | 0.5 | 0.5 | 0.5 | 0.5 | 0.5 | 0.5 | 0.5 |
| 4 | Sorbitan sesquioleate | 0.5 | 0.5 | 0.5 | 0.5 | 0.5 | 0.5 | 0.5 | 0.5 | 0.5 | 0.5 |
| 5 | Polyoxyethylene (20) hydrogenated castor oil | 0.5 | 0.5 | 0.5 | 0.5 | 0.5 | 0.5 | 0.5 | 0.5 | 0.5 | 0.5 |
| 6 | Hydrogenated lecithin | 0.1 | 0.1 | 0.1 | 0.1 | 0.1 | 0.1 | 0.1 | 0.1 | 0.1 | 0.1 |
| 7 | Triethanolamine | 0.1 | 0.1 | 0.1 | 0.1 | 0.1 | 0.1 | 0.1 | 0.1 | 0.1 | 0.1 |
| 8 | Liquid paraffin | 3 | 3 | 3 | 3 | 3 | 3 | 3 | 3 | 3 | 3 |
| 9 | Decamethylcyclopentasiloxane | 5 | 5 | 5 | 5 | 5 | 5 | 5 | 5 | 5 | 5 |
| 10 | Dimethylpolysiloxane | 3 | 3 | 3 | 3 | 3 | 3 | 3 | 3 | 3 | 3 |
| 11 | 2-Ethylhexyl p-methoxycinnamate | 3 | 3 | 3 | 3 | 3 | 3 | 3 | 3 | 3 | 3 |
| 12 | Diethylaminohydroxybenzoyl hexyl benzoate | 1 | 1 | 1 | 1 | 1 | 1 | 1 | 1 | 1 | 1 |
| 13 | Bisethylhexyloxyphenol methoxyphenyltriazine | 1 | 1 | 1 | 1 | 1 | 1 | 1 | 1 | 1 | 1 |
| 14 | Trimethylsiloxysilicic acid*1 | | | | | | | 3 | | | |
| 15 | Copolymer described in Synthesis Example 1 | 3 | | | | | | | | | |
| 16 | Copolymer described in Synthesis Example 2 | | 3 | | 8 | 0.5 | | | | | |
| 17 | Copolymer described in Synthesis Example 3 | | | 3 | | | | | | | |
| 18 | (Acrylates/VA) copolymer*2 | | | | | | | | 3 | | |
| 19 | Polyvinyl acetate*3 | | | | | | | | | 3 | |
| 20 | Acrylates copolymer*4 | | | | | | | | | | 3 |
| 21 | Purified water | Residue | Residue | Residue | Residue | Residue | Residue | Residue | Residue | Residue | Residue |

**[Table 2-2]**

| | | | | | | | | | | | |
|---|---|---|---|---|---|---|---|---|---|---|---|
| 22 | Carboxyvinyl polymer | 1 | 1 | 1 | 1 | 1 | 1 | 1 | 1 | 1 | 1 |
| 23 | 1,3-Butylene glycol | 10 | 10 | 10 | 10 | 10 | 10 | 10 | 10 | 10 | 10 |
| 24 | Ethanol | 3 | 3 | 3 | 3 | 3 | 3 | 3 | 3 | 3 | 3 |
| 25 | Phenoxyethanol | 1 | 1 | 1 | 1 | 1 | 1 | 1 | 1 | 1 | 1 |
| 26 | Microparticulate titanium oxide | 3 | 3 | 3 | 3 | 3 | 3 | 3 | 3 | 3 | 3 |
| 27 | Microparticulate zinc oxide | 2 | 2 | 2 | 2 | 2 | 2 | 2 | 2 | 2 | 2 |
| 28 | Titanium oxide | 8 | 8 | 8 | 8 | 8 | 8 | 8 | 8 | 8 | 8 |
| 29 | Iron oxide | 2.5 | 2.5 | 2.5 | 2.5 | 2.5 | 2.5 | 2.5 | 2.5 | 2.5 | 2.5 |
| 30 | Talc | 2 | 2 | 2 | 2 | 2 | 2 | 2 | 2 | 2 | 2 |
| 31 | Spherical cellulose | 0.5 | 0.5 | 0.5 | 0.5 | 0.5 | 0.5 | 0.5 | 0.5 | 0.5 | 0.5 |
| 32 | Silica | 0.5 | 0.5 | 0.5 | 0.5 | 0.5 | 0.5 | 0.5 | 0.5 | 0.5 | 0.5 |

| Evaluation item | Evaluation result | | | | | | | | | |
|---|---|---|---|---|---|---|---|---|---|---|
| (a) Water resistance of cosmetic film | ○ | ⊙ | ○ | ⊙ | ○ | × | ○ | ○ | Δ | × |
| (b) Secondary adhesion-preventing effect of cosmetic film | ○ | ⊙ | ○ | ⊙ | ○ | × | Δ | Δ | × | × |
| (c) No makeup deterioration over time | ○ | ⊙ | ○ | ⊙ | ○ | × | ○ | Δ | Δ | × |
| (d) No burden at the time of application | ⊙ | ⊙ | ⊙ | ○ | ⊙ | ⊙ | × | ○ | ○ | ○ |
| (e) No burden over time | ⊙ | ⊙ | ⊙ | ○ | ⊙ | ⊙ | × | Δ | Δ | Δ |
| (f) Stability of polymer formulation | ⊙ | ⊙ | ⊙ | ⊙ | ⊙ | - | ⊙ | ⊙ | ⊙ | ⊙ |

| | | | | | | | | | | |
|---|---|---|---|---|---|---|---|---|---|---|
| *1: KF-9021 (manufactured by Shin-Etsu Chemical Co., Ltd.) *2: VINYSOL 2140L (manufactured by Daido Chemical Corporation) *3: VINYBLAN GV-5651 (manufactured by Nissin Chemical Industry Co., Ltd.) *4: COVACRYL MS11 WP (manufactured by Sensient Cosmetic Technologies) | | | | | | | | | | |

As understood from the above table, the oil-in-water liquid foundation of each of Examples 1 to 5 was excellent in all of (a) water resistance of a cosmetic film, (b) secondary adhesion-preventing effect of a cosmetic film, (c) no makeup deterioration over time, (d) no burden at the time of application, (e) no burden over time, and (f) stability of polymer formulation. On the other hand, Comparative Example 1 in which the copolymer of the present invention was not used resulted in significantly inferior results in the items of (a) to (c). In Comparative Example 2 in which an oil-soluble silicone resin was blended instead of the copolymer of the present invention, a burden was felt at the time of application or over time. In Comparative Examples 3 to 5 in which a water-soluble polymer was blended instead of the copolymer of the present invention, all of the results were poor in the items of secondary adhesion-preventing effect and no makeup deterioration. A burden over time was felt.

### [Example 6: water-in-oil liquid foundation]

| (Component) | | (mass%) |
|---|---|---|
| 1. | Zinc laurate (3%)-treated titanium oxide | 10.0 |
| 2. | Zinc laurate (3%)-treated microparticulate titanium oxide | 1.0 |
| 3. | Zinc laurate (3%)-treated red iron oxide | 0.3 |
| 4. | Zinc laurate (3%)-treated yellow iron oxide | 1.1 |
| 5. | Zinc laurate (3%)-treated black iron oxide | 0.2 |
| 6. | Zinc laurate (3%)-treated talc | 1.0 |
| 7. | (Acrylates/ethylhexyl acrylate/dimethicone methacrylate) copolymer (*1) | 0.2 |
| 8. | PEG-9 polydimethylsiloxyethyl dimethicone (*2) | 4.0 |
| 9. | Isotridecyl isononanoate | 3.0 |
| 10. | Triethylhexanoin | 3.0 |
| 11. | 2-Ethylhexyl p-methoxycinnamate | 7.0 |
| 12. | Diethylaminohydroxybenzoyl hexyl benzoate | 1.0 |
| 13. | Isododecane | 3.0 |
| 14. | Methylphenylpolysiloxane | 3.0 |
| 15. | Dimethicone | 5.0 |
| 16. | Decamethylcyclopentasiloxane | 10.0 |
| 17. | Dimethyldistearylammonium hectorite | 1.0 |
| 18. | (Dimethicone/vinyl dimethicone) crosspolymer (*3) | 1.0 |
| 19. | Silica (average particle size 5 µm) | 1.0 |
| 20. | Polymethylsilsesquioxane (*4) | 1.0 |
| 21. | Purified water | residue |
| 22. | Ethylhexylglycerin | 0.1 |
| 23. | Phenoxyethanol | 0.3 |
| 24. | Ethanol | 5.0 |
| 25. | 1,3-Butylene glycol | 5.0 |
| 26. | Glycerin | 5.0 |
| 27. | Copolymer of Synthesis Example 2 | 3.0 |
| *1: | KP-578 manufactured by Shin-Etsu Chemical Co., Ltd. | |
| *2: | KF-6028P manufactured by Shin-Etsu Chemical Co., Ltd. | |
| *3: | KSP-102 manufactured by Shin-Etsu Chemical Co., Ltd. | |
| *4: | Tospearl 2000B manufactured by Momentive Materials Performance Japan Ltd. | |

### [Production method]

(1) Components 1 to 7 and Components 9 and 10 were mixed and treated with a three-membered roller.
(2) Component 8 and Components 11 to 20 were dispersed with a disper.
(3) (1) was added to (2) and dispersed with a disper.
(4) Components 21 to 27 were mixed and dissolved.
(5) (4) was added to (3), emulsified, and then defoamed to obtain a water-in-oil liquid foundation.

The water-in-oil liquid foundation of Example 6 was excellent in all of (a) water resistance of a cosmetic film, (b) secondary adhesion-preventing effect of a cosmetic film, (c) no makeup deterioration over time, (d) no burden at the time of application, (e) no burden over time, and (f) stability of polymer formulation.

### [Example 7: Water-based makeup base (powder-containing two-layer type)]

| (Component) | | (mass%) |
|---|---|---|
| 1. | Polyoxyethylene sorbitan trioleate | 0.5 |
| 2. | Hydrogenated lecithin | 0.5 |
| 3. | Triethanolamine | 1.0 |
| 4. | Copolymer of Synthesis Example 2 | 3.0 |
| 5. | Purified water | residue |
| 6. | Crystalline cellulose (*1) | 1.0 |
| 7. | Xanthan gum | 0.5 |
| 8. | 1,3-Butylene glycol | 5.0 |
| 9. | Dipropylene glycol | 10.0 |
| 10. | Ethanol | 5.0 |
| 11. | Phenoxyethanol | 0.3 |
| 12. | Phenylbenzimidazole sulfonic acid (*2) | 3.0 |
| 13. | Microparticulate titanium oxide | 1.0 |
| 14. | Microparticulate zinc oxide | 3.0 |
| 15. | Titanium oxide | 12.0 |
| 16. | Iron oxide | 1.0 |
| 17. | Talc | 0.5 |
| 18. | Polymethylsilsesquioxane (*3) | 1.0 |
| 19. | Polymethyl methacrylate | 1.0 |
| 20. | Silica (*4) | 0.2 |

| | | |
|---|---|---|
| *1: RHEOCRYSTA C-2SP (manufactured by DKS Co. Ltd.) *2: EUSOLEX 232 (manufactured by Merck Performance Materials) *3: Tospearl 2000B manufactured by Momentive Materials Performance Japan Ltd. *4: AEROSIL 200 (manufactured by Nippon Aerosil Co., Ltd.) | | |

### [Production method]

(1) Components 1 and 2, Components 8 and 9, and Components 13 to 20 were mixed and then treated with a three-membered roller.
(2) Components 3 to 7 and Components 10 to 12 were uniformly dissolved.
(3) (3) was added to (2), dispersed with a disper, and defoamed to obtain a water-based makeup base.

The water-based makeup base of Example 7 was excellent in all of (a) water resistance of a cosmetic film, (b) secondary adhesion-preventing effect of a cosmetic film, (c) no makeup deterioration over time, (d) no burden at the time of application, (e) no burden over time, and (f) stability of polymer formulation.

### [Example 8: oil-in-water sunscreen]

| (Component) | | (mass%) |
|---|---|---|
| 1. | (Na acrylate/Na acryloyldimethyltaurine) copolymer | 1.0 |
| 2. | Na methylstearoyl taurine (*1) | 0.5 |
| 3. | Polyoxyethylene hydrogenated castor oil | 0.3 |
| 4. | Behenyl alcohol | 0.3 |
| 5. | Inulin isostearate (*2) | 1.0 |
| 6. | Polyhydroxystearic acid | 0.5 |
| 7. | Cetyl 2-ethylhexanoate | 6.0 |
| 8. | Isotridecyl isononanoate | 4.0 |
| 9. | Ethylhexyl methoxycinnamate (*3) | 7.0 |
| 10. | Bisethylhexyloxyphenol methoxyphenyltriazine (*4) | 3.0 |
| 11. | Diethylaminohydroxybenzoyl hexyl benzoate (*5) | 2.0 |
| 12. | Polysilicone-15 (*6) | 1.5 |
| 13. | Purified water | residue |
| 14. | Xanthan gum | 0.3 |
| 15. | Carbomer (*7) | 0.5 |
| 16. | Copolymer of Synthesis Example 2 | 3.0 |
| 17. | Ethanol | 7.0 |
| 18. | Phenoxyethanol | 0.1 |
| 19. | Glycerin | 3.0 |
| 20. | 1,3-Butylene glycol | 3.0 |
| 21. | Dimethicone/octyltriethoxysilane-treated (13%) zinc oxide (*8) | 10.0 |
| 22. | Mica | 2.0 |
| 23. | Silica | 1.0 |
| 24. | (Vinyl dimethicone/methicone silsesquioxane) crosspolymer (*9) | 1.0 |
| *1: | SIMULGEL EG (manufactured by SEPPIC) | |
| *2: | Rheopearl ISK2 (manufactured by Chiba Flour Milling Co., Ltd.) | |
| *3: | Uvinul (registered trademark) MC80 (manufactured by BASF) | |
| *4: | Tinosorb (registered trademark) S (manufactured by BASF) | |
| *5: | Uvinul (registered trademark) A PLUS (manufactured by BASF) | |
| *6: | PARSOL SLX (manufactured by DSM) | |
| *7: | CARBOPOL (registered trademark) 980 (manufactured by Lubrizol Corporation) | |
| *8: | MZ-500 (manufactured by TAYCA Co., Ltd.) | |
| *9: | KSP-102 manufactured by Shin-Etsu Chemical Co., Ltd. | |

### [Production method]

(1) Components 6 to 8 and Components 21 to 24 were mixed and treated with a three-membered roller.
(2) Components 1 to 5 and Components 9 to 12 were uniformly mixed and heated to 75°C.
(3) Components 13 to 20 were uniformly mixed and heated to 75°C.
(4) (2) was added to (3), emulsified, and then cooled.
(5) (1) was added to (4), dispersed with a disper, and defoamed to obtain an oil-in-water sunscreen.

The oil-in-water sunscreen of Example 8 was excellent in all of (a) water resistance of a cosmetic film, (b) secondary adhesion-preventing effect of a cosmetic film, (c) no makeup deterioration over time, (d) no burden at the time of application, (e) no burden over time, and (f) stability of polymer formulation.

The present application is based on Japanese Patent Application No. 2021-109184 filed on June 30, 2021, the disclosure content of which is incorporated herein by reference in its entirety.

## Claims

1. A copolymer comprising more than 20 mass% and less than 50 mass% of a structural unit represented by the following Formula (1) in the copolymer, more than 0 mass% and less than 40 mass% of a structural unit represented by the following Formula (2) in the copolymer, and a structural unit represented by the following Formula (3): 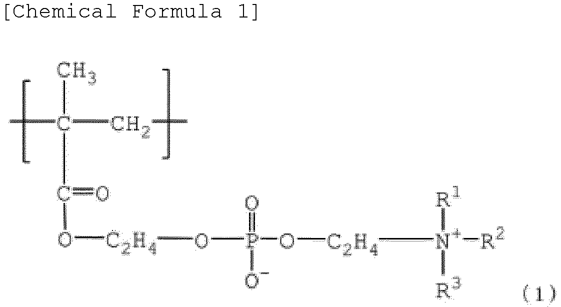 wherein R¹ to R³ each independently represent a linear or branched alkyl group having 1 to 30 carbon atoms which may be substituted, 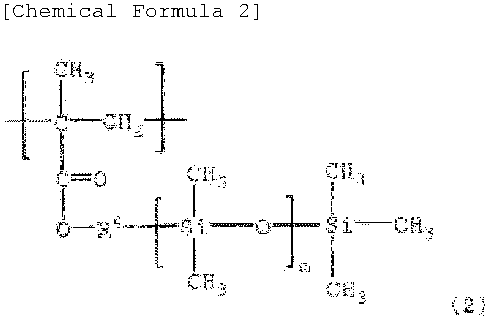 wherein R⁴ represents an alkylene group having 2 to 10 carbon atoms which may be substituted, and m represents an integer of 1 to 30, and 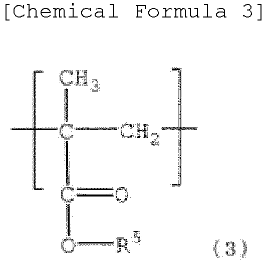 wherein R⁵ represents a linear or branched alkyl group having 1 to 30 carbon atoms which may be substituted.

2. The copolymer according to claim 1, wherein the structural unit represented by Formula (3) is contained in an amount of 20 to 70 mass% in the copolymer.

3. The copolymer according to claim 1 or 2, wherein a weight average molecular weight (Mw) is 20,000 to 100,000.

4. The copolymer according to any one of claims 1 to 3, wherein m is an integer of 8 to 16.

5. The copolymer according to any one of claims 1 to 4, wherein R¹ to R³ are each independently a linear or branched alkyl group having 1 to 3 carbon atoms which may be substituted.

6. The copolymer according to any one of claims 1 to 5, wherein a content of a structural unit other than the structural units represented by Formulas (1) to (3) is less than 1 mass% in the copolymer.

7. The copolymer according to any one of claims 1 to 6, wherein R⁵ is a linear or branched alkyl group having 1 to 8 carbon atoms which may be substituted.

8. A cosmetic comprising the copolymer according to any one of claims 1 to 7.

9. The cosmetic according to claim 8, which is a water-based or oil-in-water emulsified cosmetic.

10. The cosmetic according to claim 8 or 9, wherein a content of the copolymer is 0.01 to 20 mass%.
